# EUROPEAN PATENT SPECIFICATION

(11) **EP 0 786 936 B1**
(45) Date of publication and mention of the grant of the patent: **05.07.2000**
(21) Application number: 95935304.6
(22) Date of filing: 20.10.1995
(51) Int. Cl.: A01N 43/80, A61K 31/425

(54) **METHOD FOR REDUCING BACTERIAL CONTAMINATION IN AN AQUACULTURE**
VERFAHREN ZUR REDUKTION DER BAKTERIELLEN VERUNREINIGUNG IN DER AQUAKULTUR
PROCEDE DE REDUCTION DE LA CONTAMINATION BACTERIENNE EN AQUACULTURE

(30) Priority: 21.10.1994 EP 94203062
(43) Date of publication of application: 06.08.1997
(73) Proprietor: INVE AQUACULTURE N.V., B-9200 Dendermonde (BE)
(72) Inventor: DEHASQUE, Marleen, B-2600 Berchem (BE); DEVRESSE, Bernard, B-1080 Brussels (BE); LEGER, Philippe, B-9860 Balegem (BE); SORGELOOS, Patrick, B-9700 Oudenaarde (BE)
(74) Representative: Van Reet, Joseph
(86) International application number: BE9500096
(87) International publication number: WO9612407

(56) References cited:
- EP-A- 0 447 041
- EP-A- 0 488 606
- WO-A-84/00877
- JOURNAL OF THE WORLD AQUACULTURE SOCIETY, vol. 21, no. 2, 1990 US, pages 131-136, THOMAS A. BELL ET AL. 'A promising new chemotherapeutant for use in treatment of Fusarium solani infections in penaeid shrimp'
- DATABASE WPI Week 7126 Derwent Publications Ltd., London, GB; AN 71-44681S & JP,B,46 022 060 (TAKEDA CHEMICAL IND LTD) , 1971
- DISEASES OF AQUATIC ORGANISMS, vol. 9, no. 2, 1990 DE, pages 133-139, M.C.L. BATICADOS ET AL. 'Studies on the chemical control of luminous bacteria Vibrio harveyi and V. splendidus isolated from diseased Penaeus monodon larvae and rearing water'
- DATABASE WPI Week 9139 Derwent Publications Ltd., London, GB; AN 91-284348 & JP,A,03 187 349 (HAYASHIKANE SANGYO) , 15 August 1991

## Description

The present invention relates to a method for reducing bacterial contamination during early larval rearing in an aquaculture as a result of the addition of live food organisms thereto by treating these latter food organisms with a bactericide.

In practice, use is made for example of rotifers and subsequently of Artemia nauplii as live food organisms for aquaculture, more particularly as live food in early marine larval rearing. It is known that especially these Artemia nauplii are in practice heavily contaminated with bacteria which bloom during the hatching process and can reach numbers of more than 10⁷/g wet weight. As this bacterial flora consists mainly of Vibrio sp., potential fish/shrimp pathogens, Artemia nauplii can be considered as a possible carrier of pathogenic bacteria which can cause disease/mortality outbreaks in larval rearing, especially when the larvae are stressed.

Several studies have been carried out to reduce this bacterial load with chemotherapeutics, in particular with antibiotics. However, the use of antibiotics should be avoided due to the risks for bacterial resistance. Other, broad-spectrum chemotherapeutics, on the other hand, such as for example chlorine dioxide, were found to be very harmful for the live food organisms themselves. Reference can be made for example to the article "Susceptibility of the brine shrimp Artemia and its pathogen Vibrio parahaemolyticus to chlorine dioxide in contaminated sea-water" in the Journal of Applied Bacteriology, 1992, 73, 465-471 which discloses that the relatively low concentration of chlorine dioxide required for inactivating naturally occurring marine bacteria in sewage-contaminated sea-water destroyed Artemia nauplii and adults which are extremely sensitive to chlorine compounds, as well as other chemical compounds. This article also mentions a difference in sensitivity between the Artemia nauplii and adults.

An object of the present invention is therefore to provide a new method wherein the bacterial contamination can be effectively reduced without hampering the growth of the food organisms.

To this end, the method according to the invention is characterized in that, before being added to said aquaculture, said live food organisms are cultivated in a growing medium in the presence of a bactericidal isothiazolin derivative in an effective concentration to reduce bacterial growth therein.

It has been found that the use of a bactericidal isothiazolin derivative allows to achieve a significant reduction of bacterial numbers, especially of Vibrio sp., without negative effects on the food organism.

The intended isothiazolin derivatives are already known per se as biocides used for example in breweries for disinfecting ducts, etc. In W084/00877, isothiazolin derivatives have further already been described for use in an antimicrobial treatment process of food and fodder products. Moreover, EP-A-0 447 041 discloses the use of the intended isothiazolin derivatives as a therapeutic agent (active or preservative) in aquaculture itself. It also discloses that isothiazolones are highly effective microbicides (bactericides, fungicides and algicides). An example of the use of 5-chloro-2-methyl-4-isothiazolin-3-one and 2-methyl-4-isothiazolin-3-one in an aquaculture, more particularly a Penaeid shrimp culture, is disclosed in the article "A promising new chemotherapeutant for use in treatment of Fusarium solani infections in Penaeid shrimp" in "Journal of the world aquaculture society, Vol. 21, No. 2. The isothiazolin derivative was added to sub-adult shrimps ranging in weight from 13.5 to 14.5 g.

As to compounds included in the intended class of bactericidal isothiazolin derivatives, reference can be made to GB-A-1,224,661; US-A-3,523,121 ; US-A-3,761,488 and US-A-4,150,026 which are included herein by way of reference.

The isothiazolin derivative is preferably a derivative of 4-isothiazolin-3-one.

The invention further relates to cysts of live food organisms for aquaculture treated with a bactericide. According to the invention, said cysts, in particular Artemia cysts, are coated with a bactericidal isothiazolin derivative.

The invention also relates to an enrichment product for preparing an enrichment medium for live food organisms for an aquaculture. This enrichment product is characterized in that it comprises a bactericidal isothiazolin derivative in an effective amount to reduce bacterial growth in said enrichment medium.

Further advantages and particularities of the present invention will become apparent from the following description of some particular embodiments of the method, the cysts and the enrichment product according to the invention.

The method according to the present invention is intended to suppress bacterial contamination in aquaculture by reducing the number of bacteria associated with the live food organisms. These live food organisms are for example rotifers and Artemia but also other live food organisms can be considered. In the following part, reference will be especially made to Artemia. The live food organisms can be used for different kinds of aquacultures, in particular for the culture of Crustaceae, Mollusca and fish.

According to the invention, the live food organisms are treated with a bactericidal isothiazolin derivative. These isothiazolin derivatives are already known per se. For the particular application of the present invention, a selection should be made on the basis of the effectiveness against the bacteria associated with the live food organisms and their possible effects on these latter organisms.

A preferred class of bactericidal isothiazolin derivatives are the 4-isothiazolin-3-one derivatives. A particular preference is given to 5-chloro-2-methyl-4-isothiazolin-3-one, 2-methyl-4-isothiazolin-3-one or a combination of these two compounds.

It is also possible to use the isothiazolin derivatives as metal salt complexes described for example in US-A-4,150,026.

When used as live food organism for aquaculture, Artemia is put on the market as cysts. These cysts are hatched in a hatching medium, for example in seawater at 28°C for about 24 hours, and are usually subsequently further enriched with vitamins, fatty acids, etc. in an enrichment medium, also for about 24 hours. The enriched Artemia nauplii can then be used as live food organisms for aquaculture.

In the method according to a particular embodiment of the invention, an effective concentration of the isothiazolin derivative is provided in the grow media, i.e. in the hatching medium and if used in the enrichment medium, to reduce bacterial growth therein. Indeed, it has been observed that bacteria, including possibly pathogenic bacteria such as Vibrio sp., develop well in these media.

To suppress bacterial contamination in the hatching medium, the bactericidal isothiazolin derivative can be added directly thereto.

However, in a preferred embodiment the isothiazolin derivative is adhered to the Artemia cysts in an amount to achieve the required effective concentration in the hatching medium. In this respect, it has been found that cysts submitted to such a disinfection procedure can be stored for several months without any negative effects on the hatching efficiency and antibacterial activity.

To suppress bacterial contamination in the enrichment medium, if such a medium is used, the live food organisms are preferably first of all washed. According to the invention, a bactericidal isothiazolin derivative is further added to the enrichment medium, also in an effective amount to reduce bacterial growth therein.

In a preferred embodiment, the bactericidal isothiazolin derivative is included in an enrichment product for preparing the enrichment medium. Such an enrichment product contains for example vitamins and fatty acids and may in particular be in the form of a concentrated emulsion. An example thereof is the commercial enrichment emulsion SuperSelco from Inve aquaculture S.A., Belgium.

In both growing media, i.e. in the hatching and in the enrichment medium, the isothiazolin derivative is preferably added until a concentration between 1 and 7 ppm is achieved. This concentration is more particularly comprised between 1.5 and 4 ppm and good results have been obtained with a concentration of about 2 ppm. In case of rotifers, it may however be necessary to use much lower concentrations.

The following example illustrates the effects on the bacterial contamination of live food organisms which can be obtained by the method according to the present invention, and in particular by using cysts and an enrichment product according to the invention.

### Example

1000 g Artemia cysts (GSL-strain) where coated with 7.5 g Pastosept available from S.A. SOPURA N.V. and containing a mixture of 5-chloro-2-methyl-4-isothiazolin-3-one (10.4%) and 2-methyl-4-isothiazolin-3-one (3.5%). After drying to a humidity content of 5-8%, the cysts were vacuum packed and stored at 4°C. A same batch of cysts was kept as a control.

At different time intervals, i.e. after 2, 4, 8 and 12 weeks respectively, both the treated and control cysts were hatched in standard conditions, i.e. for 24 hours in seawater at 28°C. Two grams of cysts were added more particularly into 1 l of seawater. After 24 hours the nauplii were harvested and samples of the hatching medium and the rinsed nauplii were taken for determination of the total count on Marine agar (MA, Difco), and Vibrio sp. on TCBS (Oxoid). The hatching efficiency (number of nauplii/g) was also determined.

To test the antibacterial activity during enrichment, hatched nauplii of the disinfected and the control group were transferred to an enrichment tank and enriched with the commercial enrichment emulsion SuperSelco (Inve aquaculture S.A., Belgium). The treated Selco contained 30 g/l Pastosept and was diluted 2000 times to achieve the enrichment medium. After 24 hours the nauplii were rinsed and samples were taken for bacteriological and lipid analyses.

**Table 1**

| Number of CFU/g or /ml on MA¹and TCBS² after 24h incubation of Artemia cysts, (control and treated) and after 24h enrichment with SuperSelco (control and treated). | | |
|---|---|---|
| Treatment | MA | TCBS |
| hatching control (N)3 | 10⁸ | 10⁷ |
| hatching control (HW)⁴ | 10⁷ | 10⁶ |
| hatching disinfected (N) | 10⁴ | 10³ |
| hatching disinfected (HW) | 10³ | 10² |
| enrichment control (N) | 10⁸ | 10⁸ |
| enrichment control (EW)⁵ | 10⁷ | 10⁷ |
| enrichment disinfected (N) | 10⁵ | 10⁴ |
| enrichment disinfected (EW) | 10³ | 10³ |

| | | |
|---|---|---|
| 1 : Marine Agar - general medium for growth of marine bacteria | | |
| 2 : TCBS - specific medium for growth of Vibrio sp. | | |
| 3 : N = numbers expressed in CFU/g nauplii | | |
| 4 : HW = numbers expressed in CFU/ml hatching water | | |
| 5 : EW = numbers expressed in CFU/ml enrichment water | | |

Artemia cysts, submitted to the disinfection procedure hatched into much less contaminated nauplii compared to the control nauplii. After 24h the control nauplii contained up to 10⁶-10⁷ bacteria/g, while on the disinfected nauplii the total number was reduced to 10³-10⁴ bacteria/g. The reduction in Vibrio sp. was even more drastic (table 1).

This difference is also clearly visible in the hatching water : the hatching water after 24h hatching has a certain turbidity due to the bacterial growth, the water is completely clear when the disinfection was applied.

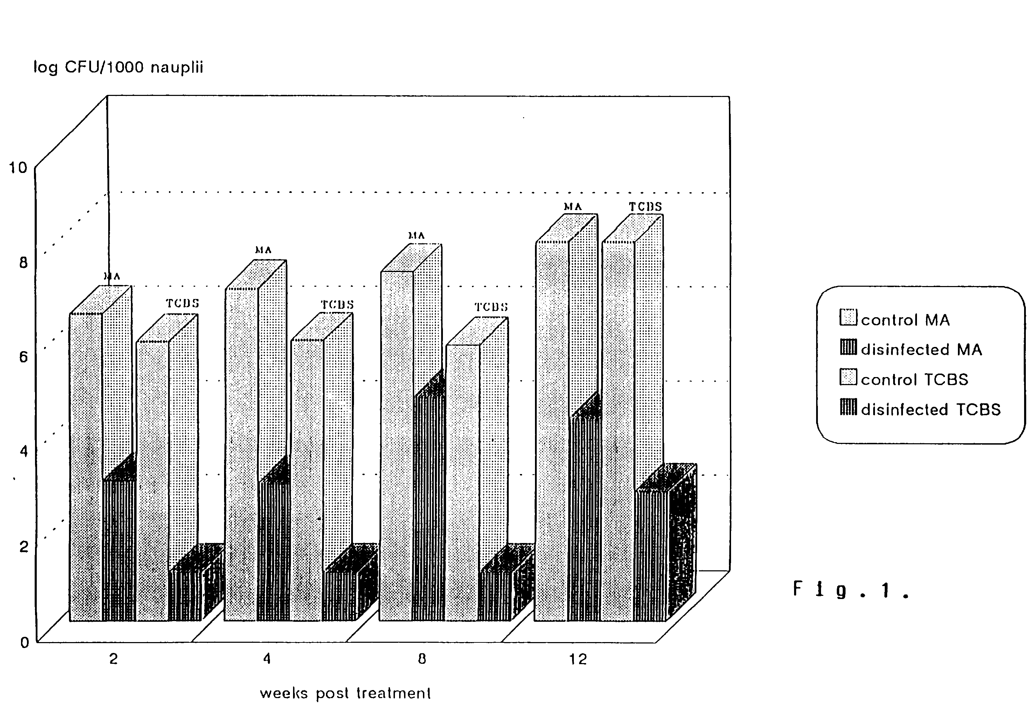

When the evolution of bacterial numbers in function of time is considered, it is clear that during a storage of 3 months at 4°C no big changes in numbers occur. The antibacterial activity is still present after that period as can be seen in Figure 1. This figure shows the evolution of bacterial numbers in function of treatment and storage period at 4°C.. There is no significant difference in hatching efficiency between control and disinfected cysts. When the disinfection procedure is also applied during enrichment, a similar reduction in bacterial numbers is observed. Still a reduction with a factor 100 to 1000 is obtained on the nauplii after 24h enrichment (table 1). The difference is even more pronounced in the enrichment water. This leads to a much "cleaner" enrichment without slime formation on the tank walls.

Lipid analyses revealed that enrichment levels with highly unsaturated fatty acids were the same in both treated and control Artemia. From this example it can be concluded that the use of a disinfection procedure during hatching and enrichment of Artemia leads to a significantly lower bacterial contamination (especially Vibrio sp.) of the nauplii. This means that the chances of transfer of potential fish pathogens via the live food to the predator is also reduced.

Cysts submitted to this disinfection procedure can be stored for several months without any negative effects on hatching efficiency and antibacterial activity.

In a preliminary feeding experiment with seabass larvae the effect of feeding the disinfected Artemia was evaluated using survival, growth and stress resistance as criteria. No negative effects could be demonstrated.

In this respect, it should be noted that in some cases, it may be preferable to add the bactericidal isothiazolin derivative only to the enrichment medium, thus without coating the Artemia cysts therewith.

## Claims

1. A method for reducing bacterial contamination during early larval rearing in an aquaculture as a result of the addition of live food organisms thereto by treating these latter food organisms with a bactericide, characterized in that, before being added to said aquaculture, said live food organisms are cultivated in a growing medium in the presence of a bactericidal isothiazolin derivative in an effective concentration to reduce bacterial growth therein.

2. A method according to claim 1, characterized in that said live food organisms are grown from cysts in a hatching medium to which said bactericidal isothiazolin derivative is added.

3. A method according to claim 2, characterized in that said isothiazolin derivative is adhered to said cysts in an amount to achieve said effective dose in the hatching medium.

4. A method according to any one of the claims 1 to 3, characterized in that said effective dose is comprised between 1 and 7 ppm, and comprises preferably 1.5 to 4 ppm, and more particularly about 2 ppm.

5. A method according to any one of the claims 1 to 4, characterized in that said live food organisms are further enriched in an enrichment medium to which said bactericidal isothiazolin derivative is added in an effective amount to reduce bacterial growth in this enrichment medium.

6. A method according to claim 5, characterized in that said bactericidal isothiazolin derivative is included in an enrichment product for preparing said enrichment medium.

7. A method according to any one of the claims 1 to 6, characterized in that said isothiazolin derivative is a derivative of 4-isothiazolin-3-one.

8. A method according to claim 7, characterized in that said isothiazolin derivative comprises 5-chloro-2-methyl-4-isothiazolin-3-one and/or 2-methyl-4-isothiazolin-3-one.

9. Cysts of live food organisms for aquaculture treated with a bactericide, characterized in that said cysts are coated with a bactericidal isothiazolin derivative.

10. Cysts according to claim 11, characterized in that said cysts are Artemia cysts.

11. Cysts according to claim 9 or 10, characterized in that said cysts are coated with an effective amount of said isothiazolin derivative to achieve an effective concentration thereof in the hatching medium to reduce bacterial growth therein.

12. An enrichment product for preparing an enrichment medium for live food organisms for an aquaculture, characterized in that said enrichment product comprises a bactericidal isothiazolin derivative in an effective amount to reduce bacterial growth in said enrichment medium.

## Patentansprüche

1. Verfahren zur Reduzierung der bakteriellen Kontamination während der frühen Larvenaufzucht in einer Aquakultur als Resultat der Hinzufügung von lebenden Nahrungsorganismen durch Behandlung dieser Nahrungsorganismen mit einem Bakterizid, dadurch gekennzeichnet, dass die erwähnten lebenden Nahrungsorganismen vor der Hinzufügung zur erwähnten Aquakultur in einem Wachstumsmedium in Anwesenheit eines bakteriziden Isothiazolinderivats in einer effektiven Konzentration aufgezogen werden, um das Bakterienwachstum darin zu reduzieren.

2. Verfahren nach Anspruch 1, dadurch gekennzeichnet, dass die erwähnten lebenden Nahrungsorganismen aus Zysten in einem Brutmedium gezogen werden, dem das erwähnte bakterizide Isothiazolinderivat zugesetzt wird.

3. Verfahren nach Anspruch 2, dadurch gekennzeichnet, dass das erwähnte Isothiazolinderivat in einer Menge an die erwähnten Zysten gebunden wird, um die erwähnte effektive Dosis im Brutmedium zu erreichen.

4. Verfahren nach einem der Ansprüche 1 bis 3, dadurch gekennzeichnet, dass die erwähnte effektive Dosis zwischen 1 und 7 ppm beträgt, und vorzugsweise zwischen 1,5 und 4 ppm liegt, und genauer insbesondere etwa 2 ppm beträgt.

5. Verfahren nach einem der Ansprüche 1 bis 4, dadurch gekennzeichnet, dass die erwähnten lebenden Nahrungsorganismen in einem angereicherten Medium weiter angereichert werden, welchem das erwähnte bakterizide Isothiazolinderivat in einer effektiven Menge beigefügt wird, um das Bakterienwachstum in diesem angereicherten Medium zu reduzieren.

6. Verfahren nach Anspruch 5, dadurch gekennzeichnet, dass das erwähnte bakterizide Isothiazolinderivat in einem Anreicherungsprodukt enthalten ist, mit dem das erwähnte angereicherte Medium zubereitet wird.

7. Verfahren nach einem der Ansprüche 1 bis 6, dadurch gekennzeichnet, dass das erwähnte Isothiazolinderivat ein Derivat des 4-Isothiazolin-3-on ist.

8. Verfahren nach Anspruch 7, dadurch gekennzeichnet, dass das erwähnte Isothiazolinderivat 5-Chloro-2-methyl-4-isothiazolin-3-on und/oder 2-Methyl-4-isothiazolin-3-on enthält.

9. Zysten von lebenden Nahrungsorganismen für Aquakultur, behandelt mit einem Bakterizid, dadurch gekennzeichnet, dass die erwähnten Zysten mit einer Schicht eines bakteriziden Isothiazolinderivats überzogen werden.

10. Zysten nach Anspruch 11, dadurch gekennzeichnet, dass die erwähnten Zysten Artemiazysten sind.

11. Zysten nach Anspruch 9 oder 10, dadurch gekennzeichnet, dass die erwähnten Zysten mit einer effektiven Menge des erwähnten Isothiazolinderivats überzogen werden, damit im Brutmedium eine effektive Konzentration davon erreicht wird, um das Bakterienwachstum darin zu reduzieren.

12. Anreicherungsprodukt für die Zubereitung eines angereicherten Mediums für lebende Nahrungsorganismen für eine Aquakultur, dadurch gekennzeichnet, dass das erwähnte Anreicherungsprodukt ein bakterizides Isothiazolinderivat in einer effektiven Menge enthält, um das Bakterienwachstum im erwähnten angereicherten Medium zu reduzieren.

## Revendications

1. Procédé de réduction d'une contamination bactérienne au cours d'un élevage larvaire précoce dans une aquaculture à la suite de l'addition d'organismes alimentaires vivants à celle-ci en traitant ces derniers organismes alimentaires avec un bactéricide, caractérisé en ce que, avant d'être ajoutés à ladite aquaculture, lesdits organismes alimentaires vivants sont cultivés dans un milieu de croissance en présence d'un dérivé d'isothiazoline bactéricide en une concentration efficace pour y réduire la croissance bactérienne.

2. Procédé suivant la revendication 1, caractérisé en ce que les organismes alimentaires vivants susdits sont mis à croître à partir de vésicules dans un milieu d'incubation auquel ledit dérivé d'isothiazoline bactéricide est ajouté.

3. Procédé suivant la revendication 2, caractérisé en ce qu'on fait adhérer le dérivé d'isothiazoline à ces vésicules en une quantité permettant d'atteindre la dose efficace précitée dans le milieu d'incubation.

4. Procédé suivant l'une quelconque des revendications 1 à 3, caractérisé en ce que ladite dose efficace est comprise entre 1 et 7 ppm, et est avantageusement de 1,5 à 4 ppm, et plus particulièrement d'environ 2 ppm.

5. Procédé suivant l'une quelconque des revendications 1 à 4, caractérisé en ce que les organismes alimentaires vivants sont de plus enrichis dans un milieu d'enrichissement auquel le dérivé d'isothiazoline bactéricide est ajouté en une quantité efficace pour réduire la croissance bactérienne dans ce milieu d'enrichissement.

6. Procédé suivant la revendication 5, caractérisé en ce que le dérivé d'isothiazoline bactéricide est incorporé dans un produit d'enrichissement pour préparer le milieu d'enrichissement précité.

7. Procédé suivant l'une quelconque des revendications 1 à 6, caractérisé en ce que le dérivé d'isothiazoline est un dérivé de 4-isothiazolin-3-one.

8. Procédé suivant la revendication 7, caractérisé en ce que le dérivé d'isothiazoline comprend de la 5-chloro-2-méthyl-4-isothiazolin-3-one et/ou de la 2-méthyl-4-isothiazolin-3-one.

9. Vésicules d'organismes alimentaires vivants pour l'aquaculture traitées avec un bactéricide, caractérisées en ce que lesdites vésicules sont recouvertes d'un dérivé d'isothiazoline bactéricide.

10. Vésicules suivant la revendication 9, caractérisées en ce que lesdites vésicules sont des vésicules d'artémia.

11. Vésicules suivant l'une ou l'autre des revendications 9 et 10, caractérisées en ce que lesdites vésicules sont recouvertes d'une quantité efficace du dérivé d'isothiazoline précité pour obtenir une concentration efficace de celui-ci dans le milieu d'incubation de manière à y réduire la croissance bactérienne.

12. Produit d'enrichissement pour préparer un milieu d'enrichissement pour organismes alimentaires vivants pour une aquaculture, caractérisé en ce que ledit produit d'enrichissement comprend un dérivé d'isothiazoline bactéricide en une quantité efficace pour réduire la croissance bactérienne dans ledit milieu d'enrichissement.
